(19) Europäisches Patentamt
European Patent Office
Office européen des brevets

(11) Veröffentlichungsnummer: **0 017 907**
**B1**

# (12) EUROPÄISCHE PATENTSCHRIFT

(45) Veröffentlichungstag der Patentschrift: **09.02.83**

(51) Int. Cl.³: **C 25 B 3/02** // C07C175/00

(21) Anmeldenummer: **80101892.0**

(22) Anmeldetag: **09.04.80**

(54) **Verfahren zur Herstellung von Cyclohexenderivaten.**

(30) Priorität: **12.04.79 US 29170**

(43) Veröffentlichungstag der Anmeldung: **29.10.80 Patentblatt 80/22**

(45) Bekanntmachung des Hinweises auf die Patenterteilung: **09.02.83 Patentblatt 83/6**

(84) Benannte Vertragsstaaten: **AT BE CH DE FR GB IT LI NL**

(56) Entgegenhaltungen:
**US-A-3 448 022**
**US-A-4 098 827**
**J. CHEM. SOC. (C), 1968, Seiten 2055–2059 A.J. BAGGALEY et al.: »Anodic Oxidation. Part V Some Reactions with Cyclohexa-1,3-diene and Cyclohexene«**

(73) Patentinhaber: **F. HOFFMANN-LA ROCHE & CO. Aktiengesellschaft, CH-4002 Basel (CH)**

(72) Erfinder: **Hengartner, Urs, 28 Binzenstrasse, CH-4058 Basel (CH)**
Erfinder: **Reymond, Gilbert, 4 Spruce Road, Clinton, N.J. (US)**
Erfinder: **Toome, Valdemar, 18 Carrie Court, Nutley, N.J. (US)**

(74) Vertreter: **Lederer, Franz, Dr. et al, Patentanwälte Dr. Franz Lederer Reiner F. Meyer Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Verfahren zur Herstellung von Cyclohexenderivaten

Die vorliegende Erfindung betrifft ein neues Verfahren zur Herstellung von Zwischenprodukten in der Gesamtsynthese von Canthaxanthin.

Das Verfahren der vorliegenden Erfindung ist dadurch gekennzeichnet, daß man zur Herstellung einer Verbindung der Formel

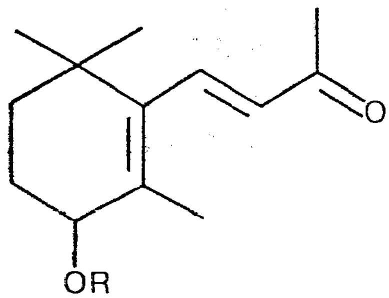

II

worin R Wasserstoff oder $C_1-C_7$-Alkanoyl bedeutet,

eine Lösung der Verbindung der Formel

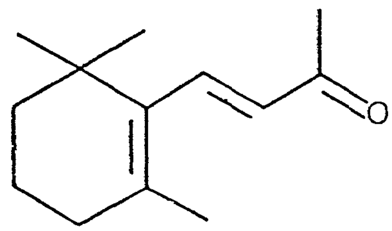

I

in einer $C_1-C_7$-Alkansäure in Gegenwart eines Elektrolyten elektrolysiert und daß man, gewünschtenfalls, eine so erhaltene Verbindung der allgemeinen Formel II, worin R $C_1-C_7$-Alkanoyl bedeutet, zur Verbindung der Formel III

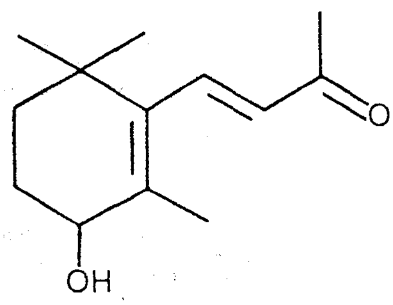

III

hydrolysiert.

Im Rahmen der vorliegenden Erfindung bedeutet der Ausdruck »Niederalkanoyl« niedere Alkanoyl-Gruppen mit 1 bis 7 Kohlenstoffatomen, wie Formyl, Acetyl, Propionyl, Butyryl, 2-Methylbutyryl, Pentanoyl, Hexanoyl und dergleichen. Der Ausdruck »Niederalkansäure« bedeutet Alkansäuren mit 1 bis 7 Kohlenstoffatomen, wie Ameisensäure, Essigsäure, Propionsäure, Buttersäure, 2-Methylbuttersäure, Pentansäure, Hexansäure und dergleichen. Der Ausdruck »Niederalkyl« bezeichnet gesättigte, geradkettige und verzweigtkettige, aliphatische Kohlenwasserstoffreste mit 1 bis 7 Kohlenstoffatomen wie Methyl, Äthyl, Isopropyl, n-Propyl und dergleichen.

Gemäß US-A-4 098 827 wurde Hydroxy-$\beta$-ionon der Formel III, ein bekanntes Zwischenprodukt für Canthaxanthin, aus $\alpha$-Ionon durch Epoxidierung und Ringöffnung hergestellt. Obwohl dies ein leistungsfähiges Verfahren darstellt, wäre es wünschenswert, daß die Verbindung der Formel III aus einem Ausgangsmaterial, das leichter zugänglich ist als $\alpha$-Ionon, beispielsweise aus $\beta$-Ionon, hergestellt werden könnte.

In der Vergangenheit wurden Monoolefine elektrochemisch acyloxyliert (US-A-3 448 022). Dieses Verfahren war brauchbar für Monoolefine wie Cyclohexen; es wurde jedoch nicht auf Polyolefine angewandt, geschweige denn auf Polyolefine mit einem Oxosubstituenten.

Gemäß der vorliegenden Erfindung wurde nun gefunden, daß Alkanoyloxylierung selektiv in der

3-Stellung des Cyclohex-1-en-Ringes in Ausbeuten von mindestens 48% stattfindet, wenn ein Diolefin mit einer Ketogruppe, wie die Verbindung der Formel I einer elektrochemischen Alkanoyloxylierung unterworfen wird.

Die elektrochemische Reaktion des erfindungsgemäßen Verfahrens wird unter Verwendung eines Lösungsmittels und eines Elektrolyten in einer elektrochemischen Zelle ausgeführt, welche durch eine semipermeable Membran in einen Anoden- und einen Kathodenteil unterteilt ist. Zur Durchführung dieser Reaktion wird die Verbindung der Formel I in den Anodenteil eingebracht und an die Zelle eine elektrische Spannung angelegt. Die Reaktion liefert im Anodenteil der Zelle die Verbindung der Formel II, worin R = Niederalkanoyl, und als Nebenprodukt eine kleine Menge der Verbindung der allgemeinen Formel

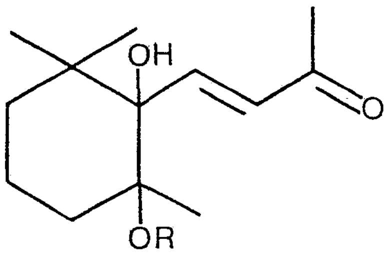

IV

worin R die obige Bedeutung hat.

In einer bevorzugten Ausführungsform der vorliegenden Erfindung wird als Elektrolyt Tetra(niederalkyl)ammonium-p-toluolsulfonat, besonders bevorzugt Tetraäthylammonium-p-toluolsulfonat verwendet. Dadurch wird die Ausbeute der Verbindung der Formel II, worin R = Niederalkanoyl, wesentlich erhöht und folglich die Menge der Verbindung der Formel IV vermindert.

Die erfindungsgemäße Umsetzung der Verbindung der Formel I zur Verbindung der Formel II, worin R = Niederalkanoyl, kann nach irgendeiner gebräuchlichen Elektrolysenmethode und in irgendeiner der üblicherweise verwendeten elektrolytischen Zellen erfolgen. Die Reaktion wird in Gegenwart eines Lösungsmittels und eines Elektrolyten ausgeführt.

Als Lösungsmittelmedium für die Elektrolyse werden Niederalkansäuren verwendet. Die Wahl der zu verwendenden Niederalkansäuren wird durch die gewünschte Alkanoylgruppe R in der Verbindung der Formel II bestimmt. Zu den bevorzugten Alkansäuren gehören Essigsäure, Propionsäure, Buttersäure, 2-Methylbuttersäure und Pentansäure. Besonders bevorzugt ist Essigsäure. Im allgemeinen werden diese Säuren in wasserfreiem Zustand oder mit einer Menge von bis zu etwa 5 Gewichtsprozent Wasser verwendet. Diese Menge ist jedoch nicht kritisch, da auch namhafte Mengen Wasser verwendet werden können.

Als Elektrolyt kann gemäß der vorliegenden Erfindung irgendein in Elektrolyseverfahren üblicherweise verwendeter Elektrolyt benützt werden. Bevorzugte Elektrolyten sind Ammonium- oder Alkalimetallsalze oder Kobaltsalze der als Lösungsmittel verwendeten Säure. Besonders bevorzugte Elektrolyten sind gemäß der bevorzugten Ausführungsform der vorliegenden Erfindung die Tetra(niederalkyl)ammonium-p-toluolsulfonate. Der Elektrolyt liegt in der Lösung zweckmäßig in einer Menge von etwa 0,5 bis etwa 10 Gewichtsprozent der als Lösungsmittel verwendeten Niederalkancarbonsäure vor. In der bevorzugten Ausführungsform enthält der Elektrolyt vorzugsweise etwa 40 bis etwa 10 Gewichtsprozent Tetra(niederalkyl)ammonium-p-toluolsulfonat, wobei der restliche Teil ein herkömmlicher Elektrolyt ist, beispielsweise ein Alkalimetallsalz oder ein quaternäres Ammoniumsalz der Niederalkansäure.

Die Elektroden können aus Kohlenstoff, Graphit oder irgendeinem inerten Metall, wie Kupfer, Palladium, rostfreiem Stahl, Platin, Silber, Chrom, Nickel, Blei oder Gold bestehen. Die Kathode besteht vorzugsweise aus Kohlenstoff, Platin, Palladium, Nickel, Chrom oder Gold. Um sehr gute Resultate zu erhalten, wird bevorzugt eine Anode aus Kohlenstoff oder Graphit verwendet. Zur Trennung des Anoden- und Kathodenteils der Zelle kann irgendeine der üblicherweise verwendeten semipermeablen Membranen eingesetzt werden. Diese Maßnahme dient dazu, mögliche Reaktionen der Reaktionsprodukte an der Anode mit Produkten an der Kathode zu verhindern. Bevorzugte Membranmaterialien sind Porzellan, Glasfritte, Polyäthylen und dergleichen. Zur Durchführung der Reaktion wird mittels einer üblichen Spannungsquelle eine Spannung von etwa 5 bis etwa 500 Volt angelegt.

Die Stromdichte bestimmt die Reaktionsgeschwindigkeit der Elektrolyse. Sie kann über einen ziemlich breiten Bereich variiert werden. Zweckmäßigerweise liegt die Stromdichte zwischen etwa 0,001 und etwa 5 $A/cm^2$, vorzugsweise zwischen etwa 0,01 und etwa 0,5 $A/cm^2$. Die Temperatur ist kein kritischer Aspekt in dieser Reaktion. Die Reaktion kann sowohl bei Raumtemperatur als auch bei höherer oder tieferer Temperatur ausgeführt werden. Im allgemeinen wird eine Reaktionstemperatur von etwa 20 bis etwa 40° C bevorzugt. Es kann jedoch irgendeine Temperatur angewendet werden, die

nicht höher als der Siedepunkt der Lösung ist.

Die Reaktionsdauer kann je nach Stromdichte 1 bis 480 Stunden oder mehr betragen. Die Verbindung der Formel II wird im Anodenteil der Zelle gebildet. Sie kann nach bekannten Methoden, beispielsweise durch Extraktion, aus der Lösung im Anodenteil isoliert werden.

Die Verbindung der Formel II, worin R = Niederalkanoyl, kann durch Hydrolyse zur Verbindung der Formel III umgesetzt werden. Diese Umsetzung kann nach irgendeinem bekannten Hydrolyseverfahren erfolgen.

Die vorliegende Erfindung wird durch die folgenden Beispiele weiter veranschaulicht. Die beschriebenen Reaktionen wurden in einer unterteilten Zelle bei einer Stromdichte von 0,0022 A/$cm^2$ ausgeführt. Als Anode wurde ein Kohlenstoffstab, als Kathode ein Platindraht und als Elektrolyt Tetraäthylammonium-p-toluolsulfonat (TEATS) oder Natriumacetat verwendet. Die Unterteilung der Zelle in einen Anoden- und einen Kathodenteil erfolgte durch eine Membran aus Glasfritte. Der Anodenteil bestand aus einem Glasgefäß mit 6,3 cm (2½ inches) Durchmesser, 8,9 cm (3½ inches) Länge und einem Fassungsvermögen von 200 ml. Der Kathodenteil war ein Glasrohr mit 2,5 cm (1 inch) Durchmesser und 10,2 cm (4 inches) Länge, welches mit dem Anodengefäß über eine Standardverbindung verbunden war.

## Beispiel 1

0,946 g β-Ionon wurden anodischer Acetoxylierung unterworfen (0,3 M Natriumacetat in Essigsäure, 2 mA, 124 Stunden). Anschließend wurde die Reaktionslösung in Methylenchlorid/Wasser aufgenommen, die Methylenchlorid-Phase mit Wasser, gesättigter Natriumbicarbonat-Lösung und Salzwasser gewaschen und über Natriumsulfat getrocknet. Nach dem Einengen am Rotationsverdampfer wurden 1,05 g eines Öls erhalten. 996 mg des Rohproduktes wurden auf 150 g Silicagel mit Chloroform/Äthylacetat 10 : 1 bzw. 5 : 1 chromatographiert und die folgenden Produkte isoliert und analysiert:

101 mg (11%) β-Ionon
390 mg (33%) Acetoxy-β-ionon
274 mg (22%) der Verbindung der Formel IV,

worin R Acetyl bedeutet. Die analytische Probe dieser Verbindung wurde durch Destillation und Kristallisation aus Hexan hergestellt. Dabei wurden weiße Nadeln mit Smp. 84,5 – 85,5° C erhalten.

## Beispiel 2

0,932 g β-Ionon wurden anodischer Acetoxylierung unterworfen (0,3 M Tetraäthylammonium-p-toluolsulfonat [TEATS] in Essigsäure, 50 mA, 8,5 Stunden). Anschließend wurde die braune Reaktionslösung am Rotationsverdampfer auf ein Volumen von 10 ml eingeengt und in Methylenchlorid/Wasser aufgenommen. Die Methylenchlorid-Phase wurde mit gesättigter Natriumbicarbonat-Lösung und Salzwasser gewaschen, die wäßrigen Phasen wurden nochmals mit Methylenchlorid extrahiert und die vereinigten organischen Phasen über Natriumsulfat getrocknet. Durch Einengen am Rotationsverdampfer wurden 1,23 g rohes Acetoxy-β-ionon (Reinheit 53,5% nach Gaschromatographieanalyse) erhalten. 1,18 g dieses Rohmaterials wurden bei Raumtemperatur mit Natriumhydroxid in Methanol/Wasser (20 : 3 v/v) verseift. Nach 1 Stunde wurde das Reaktionsgemisch mit Essigsäure angesäuert und am Rotationsverdampfer eingeengt. Der Rückstand wurde in Toluol/Wasser aufgenommen und die Toluol-Phase mit gesättigter, wäßriger Natriumbicarbonat-Lösung gewaschen. Die wäßrigen Phasen wurden nochmals mit Toluol extrahiert und die vereinigten Extrakte über Natriumsulfat getrocknet. Nach dem Einengen am Vakuum wurden 826 mg rohes Hydroxy-β-ionon erhalten, das nach Destillation (105 – 120° C/0,1 mm Hg) 597 mg (62%) Hydroxy-β-ionon als gelbes, viskoses Öl ergab (Reinheit 57,9% gemäß Gaschromatographieanalyse).

## Beispiele 3 – 8

In den Beispielen 3 bis 8 wurde jeweils 1 ml 93%iges β-Ionon nach der in den Beispielen 1 und 2 beschriebenen Methode und unter den in der Tabelle angegebenen Bedingungen einer Elektrolyse unterworfen. In der Tabelle bezeichnen II und IV die Verbindungen der Formeln II und IV, worin R Acetyl bedeutet, und I bezeichnet nicht-umgesetztes β-Ionon der Formel I. Die aufgeführten Ausbeuten wurden durch Dampfphasenchromatographie des rohen Reaktionsgemisches bestimmt.

Tabelle

Übersicht über die experimentellen Bedingungen

| Beispiel | Elektrolyt | Strom | Reaktionszeit | Ausbeute |
|---|---|---|---|---|
| 3 | 0,3 M Natriumacetat in Eisessig | 2,2 mA | 153 h | 60% II<br>28% IV<br>9% I |
| 4 | 0,3 M Natriumacetat in Eisessig | 2,0 mA | 124 h | 49% II<br>22% IV |
| 5 | 0,3 M Natriumacetat in 98%iger Essigsäure | 5,0 mA | 52 h | 47% II<br>23% IV<br>18% I |
| 6 | 0,3 M Natriumacetat in Eisessig | 2,0 mA | 216 h | 56% II<br>20% IV<br>5% I |
| 7 | 0,3 M TEATS in Eisessig | 50,0 mA | 5 h 40′ | 65% II<br>24% I |
| 8 | 0,3 M TEATS in Eisessig | 50,0 mA | 8 h 30′ | 54% II<br>18% I<br>28% verschiedene nichtidentifizierte Nebenprodukte |

**Patentansprüche**

1. Verfahren zur Herstellung einer Verbindung der Formel

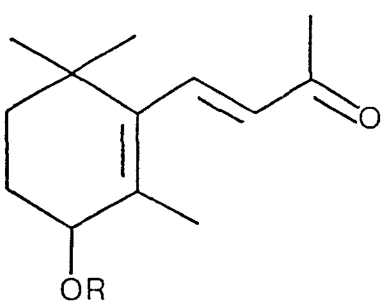

II

worin R Wasserstoff oder $C_1-C_7$-Alkanoyl bedeutet,

dadurch gekennzeichnet, daß man eine Lösung der Verbindung der Formel

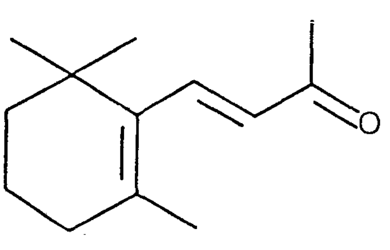

I

in einer $C_1-C_7$-Alkansäure in Gegenwart eines Elektrolyten elektrolysiert und daß man, gewünschtenfalls, eine so erhaltene Verbindung der allgemeinen Formel II, worin R $C_1-C_7$-Alkanoyl bedeutet, zur Verbindung der Formel II, worin R Wasserstoff bedeutet, hydrolysiert.

2. Verfahren nach Anspruch 1, dadurch gekennzeichnet, daß man als Anode eine Kohlenstoffelektrode verwendet.

3. Verfahren nach Anspruch 1 oder 2, dadurch gekennzeichnet, daß man einen Elektrolyten verwendet, der Tetra($C_1-C_7$-alkyl)ammonium-p-toluolsulfonat enthält.

4. Verfahren nach Anspruch 3, dadurch gekennzeichnet, daß man einen Elektrolyten verwendet, der Tetraäthylammonium-p-toluolsulfonat enthält.

5. Verfahren nach Anspruch 3 oder 4, dadurch gekennzeichnet, daß man einen Elektrolyten verwendet, der 40 bis 100 Gewichtsprozent Tetra($C_1-C_7$-alkyl)ammonium-p-toluolsulfonat enthält.

6. Verfahren nach einem der Ansprüche 1 bis 5, dadurch gekennzeichnet, daß der Elektrolyt in einer Menge von 0,5 bis 10 Gewichtsprozent der Niederalkancarbonsäure verwendet wird.

7. Verfahren nach einem der Ansprüche 1 bis 6, dadurch gekennzeichnet, daß die Elektrolyse bei einer Stromdichte von 0,001 bis 5 A/cm², vorzugsweise 0,01 bis 0,5 A/cm² ausgeführt wird.

8. Verfahren nach einem der Ansprüche 1 bis 7, dadurch gekennzeichnet, daß man als Lösungsmittelmedium Essigsäure, Propionsäure, Buttersäure, 2-Methylbuttersäure oder Pentansäure verwendet.

9. Verfahren nach Anspruch 8, dadurch gekennzeichnet, daß man als Lösungsmittelmedium Essigsäure verwendet.

10. Verfahren nach einem der Ansprüche 1 bis 9, dadurch gekennzeichnet, daß man die Elektrolyse bei einer Temperatur ausführt, die nicht höher als der Siedepunkt des Reaktionsgemisches, vorzugsweise 20 bis 40° C, ist.

**Claims**

1. Process for the manufacture of a compound of the formula

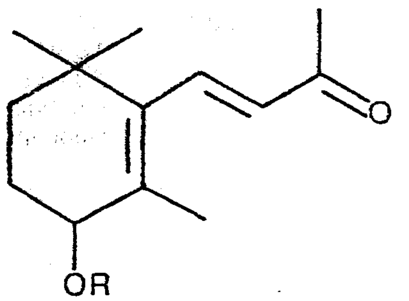

II

wherein R signifies hydrogen or $C_1-C_7$-alkanoyl,

characterized by electrolyzing a solution of the compound of the formula

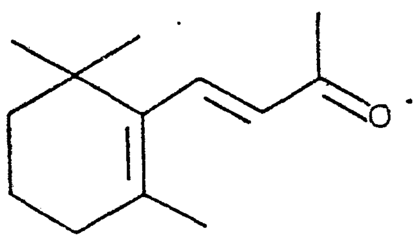

I

in a $C_1-C_7$-alkanoic acid in the presence of an electrolyte and, if desired, hydrolyzing a thus-obtained compound of general formula II wherein R signifies $C_1-C_7$-alkanoyl to the compound of formula II wherein R signifies hydrogen.

2. Process according to claim 1, characterized in that a carbon electrode is used as the anode.

3. Process according to claim 1 or 2, characterized in that an electrolyte which contains tetra($C_1-C_7$-alkyl)-ammonium p-toluenesulphonate is used.

4. Process according to claim 3, characterized in that an electrolyte which contains tetraethylammonium p-toluenesulphonate is used.

5. Process according to claim 3 or 4, characterized in that an electrolyte which contains 40 to 100 weight percent of tetra($C_1-C_7$-alkyl)ammonium p-toluenesulphonate is used.

6. Process according to any one of claims 1 to 5, characterized in that the electrolyte is used in an amount of 0.5 to 10 weight percent of the lower alkanecarboxylic acid.

7. Process according to any one of claims 1 to 6, characterized in that the electrolysis is carried out at a current density of 0.001 to 5 A/cm², preferably 0.01 to 0.5 A/cm².

8. Process according to any one of claims 1 to 7, characterized in that acetic acid, propionic acid, butyric acid, 2-methylbutyric acid or pentanoic acid is used as the solvent medium.

9. Process according to claim 8, characterized in that acetic acid is used as the solvent medium.

10. Process according to any one of claims 1 to 9, characterized in that the electrolysis is carried out at a temperature which is not higher than the boiling point of the reaction mixture, preferably 20 to 40° C.

**Revendications**

1. Procédé de préparation d'un composé de formule

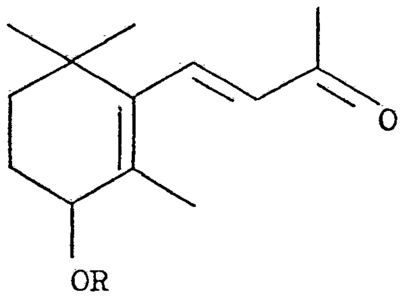

II

dans laquelle R représente l'hydrogène ou un groupe alcanoyle en C1 – C7,

caractérisé en ce que l'on électrolyse une solution du composé de formule

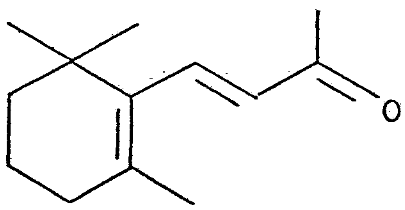

I

dans un acide alcanoïque en C1 – C7 en présence d'un électrolyte et, si on le désire, on convertit un composé obtenu, répondant à la formule générale II dans laquelle R représente un groupe alcanoyle en C1 – C7 en le composé de formule II dans laquelle R représente l'hydrogène par hydrolyse.

2. Procédé selon la revendication 1, caractérisé en ce que l'on utilise en tant qu'anode une électrode de carbone.

3. Procédé selon la revendication 1 ou 2, caractérisé en ce que l'on utilise un électrolyte qui contient un p-toluène-sulfonate de tétra-(alkyle en C1 – C7)-ammonium.

4. Procédé selon la revendication 3, caractérisé en ce que l'on utilise un électrolyte qui contient du p-toluène-sulfonate de tétraéthylammonium.

5. Procédé selon la revendication 3 ou 4, caractérisé en ce que l'on utilise un électrolyte qui contient de 40 à 100% en poids d'un p-toluène-sulfonate de tétra-(alkyle en C1 – C7)-ammonium.

6. Procédé selon l'une des revendications 1 à 5, caractérisé en ce que l'on utilise l'électrolyte en quantité de 0,5 à 10% du poids de l'acide alcanecarboxylique inférieur.

7. Procédé selon l'une des revendications 1 à 6, caractérisé en ce que l'on effectue l'électrolyse à une densité de courant de 0,001 à 5 A/cm$^2$, de préférence de 0,01 à 0,5 A/cm$^2$.

8. Procédé selon l'une des revendications 1 à 7, caractérisé en ce que l'on utilise en tant que milieu solvant l'acide acétique, l'acide propionique, l'acide butyrique, l'acide 2-méthylbutyrique ou l'acide pentanoïque.

9. Procédé selon la revendication 8, caractérisé en ce que l'on utilise en tant que milieu solvant l'acide acétique.

10. Procédé selon l'une des revendications 1 à 9, caractérisé en ce que l'on effectue l'électrolyse à une température qui ne dépasse pas le point d'ébullition du mélange de réaction et qui est de préférence de 20 à 40° C.